# EUROPÄISCHE PATENTANMELDUNG

(11) **EP 1 795 605 A1**
(43) Veröffentlichungstag der Anmeldung: **13.06.2007**
(21) Anmeldenummer: 05027062.8
(22) Anmeldetag: 12.12.2005
(51) Int. Cl.: C12Q 1/25, G01N 1/34, G01N 33/52

(54) **Chromogene Tests in Gegenwart von Zellen**

(71) Anmelder: Roche Diagnostics GmbH, 68305 Mannheim (DE); F. Hoffmann-La Roche AG, 4070 Basel (CH)
(72) Erfinder: Watzele, Manfred, 82362 Weilheim (DE); Nikolaus, Thomas, 81475 München (DE); Rode, Hans-Jürgen, 69181 Leimen-St. Ilgen (DE)

(57) **Zusammenfassung**

Die vorliegende Erfindung betrifft ein Verfahren zur colorimetrischen Messung von Enzymaktivitäten, enthaltend folgende Schritte
- Bereitstellen einer Zellsuspension oder eines Zellbestandteile enthaltenden Zell-Lysates
- Zugabe eines chromogenen Substrates zu Beginn der Enzymreaktion
- Zugabe einer ersten weiteren Substanz zum Abstoppen der Enzymreaktion,
- Colorimetrische Messung

wobei während oder nach Beendigung der Enzymreaktion eine zweite weitere Substanz zugesetzt wird, die die durch die Zellen oder die Zellbestandteile verursachte Lichtstreuung und/oder Absorption minimieren.

## Beschreibung

Die vorliegende Erfindung entstammt dem Gebiet der colorimetrischen Messung von Enzymaktivitäten in zellulären Suspensionen oder Lysaten. Insbesondere betrifft die vorliegende Erfindung ein Verfahren zur Messung von Enzymaktivitäten, bei denen die durch Zellbestandteile verursachte Trübung der Probe durch Zugabe geeigneter Additive minimiert wird.

### Stand der Technik

Will man die Wirkung von Reagenzien, Wirkstoffen, oder anderer Einflüsse auf einen Organismus prüfen, so werden diese Experimente oft in in vitro Modellen in der Zellkultur durchgeführt. Die verschiedenen enzymatischen Aktivitäten der Zellen geben dabei Aufschluss über deren Zustand und physiologische Eigenschaften.

Häufig versucht man die Aktivierung von Genen durch sogenannte Reportergen-Tests nachzuweisen. Dabei wird das Gen für ein bestimmtes Nachweisenzym (=Reportergen) hinter einen Promoter gesetzt, dessen Aktivierung man nachweisen will. Als Reportergene können hierbei auch sezernierte Enzyme wie beispielsweise die sekretorische Alkalische Phosphatase eingesetzt werden, deren Aktivität man dann mit dem Enzymsubstrat 4-Nitrophenylphosphat testet. Dieses Substrat wird dabei von diesem Enzym zum Produkt 4-Nitrophenol umgesetzt, welches bei der Wellenlänge von 405 nm Licht absorbiert.

Will man Zellen genetisch verändern, so muss man die entsprechende Plasmid-DNA mit Hilfe geeigneter Verfahren zunächst durch die Plasmamembran und dann in den Zellkern befördern. Die dabei angewendeten Verfahren wie beispielsweise die Lipofektion müssen dabei jeweils für die entsprechende Zellinie optimiert werden. Auch in diesen Fällen, wo man die Transfizierbarkeit von unterschiedlichen Zellen optimieren will, werden häufig Plasmide mit Reportergenen wie beispielsweise der sekretorischen alkalischen Phosphatase eingesetzt. Man kann dann an der gemessenen Enzymaktivität den Erfolg der Transfektion überprüfen.

Sehr häufig werden auch unterschiedliche Methoden zur Messung von zytotoxischen Eigenschaften eines Stoffes angewendet. Eine Reihe von Tests zur Zytotoxizitätsbestimmung wird in der Literatur gefunden.

Viele Tests hierzu basieren auf der Eigenschaft zytotoxischer Stoffe die Zellmembran zu schädigen. Dabei werden von Zellen deren Plasmamembran geschädigt wurde Enzyme freigesetzt, welche im Zellkulturüberstand über einen enzymatischen Test nachgewiesen werden können. Die Menge der freigesetzten Enzyme ist dabei proportional zur Anzahl der geschädigten Zellen. Derartige Enzym-Freisetzungstests wurden für die Glutamat-Oxalacetat Transaminase, für die Glutamat Pyruvat Transaminase, für die Arginosuccinat Lyase und für alkalische und saure Phosphatase beschrieben (Masanet, J., Gomez-Lechon, M. J., und Castell, J. V., Toxic. in Vitro 2 (1988) 275-282; Martin, Angela, und Clynes, Martin, In Vitro Cell Dev. Biol. 27A (1991) 183-184). Auf diese Weise wurde beispielsweise die von Lymphozyten hervorgerufene Freisetzung alkalischer Phosphatase aus humanen embryonischen Fibroblasten getestet (Szekeres, Julia, Pacsa, A.S., und Pejtsik, B., J. Immun. Meth. 40 (1981) 151-154). Am häufigsten jedoch wird für die Enzym-Freisetzungstests die Lactatdehydrogenase eingesetzt, da die Bestimmung der vorher genannten Enzyme oft durch deren geringe Menge in vielen Zellen erschwert wird. Im Gegensatz zu den anderen Enzymen ist die Lactatdehydrogenase ein sehr stabiles cytoplasmatisches Enzym, welches in allen Zellen vorhanden ist. Es wird von Zellen mit geschädigter Plasmamembran sehr schnell freigesetzt und kann leicht im Kulturüberstand nachgewiesen werden (Decker, Thomas, und Lohmann-Matthes, Marie-Luise, J. Immun. Meth. 15 (1988) 61-69; Korzeniewski, Carol, und Callewaert, Denis M., J. Immun. Meth. 64 (1983) 313-320).

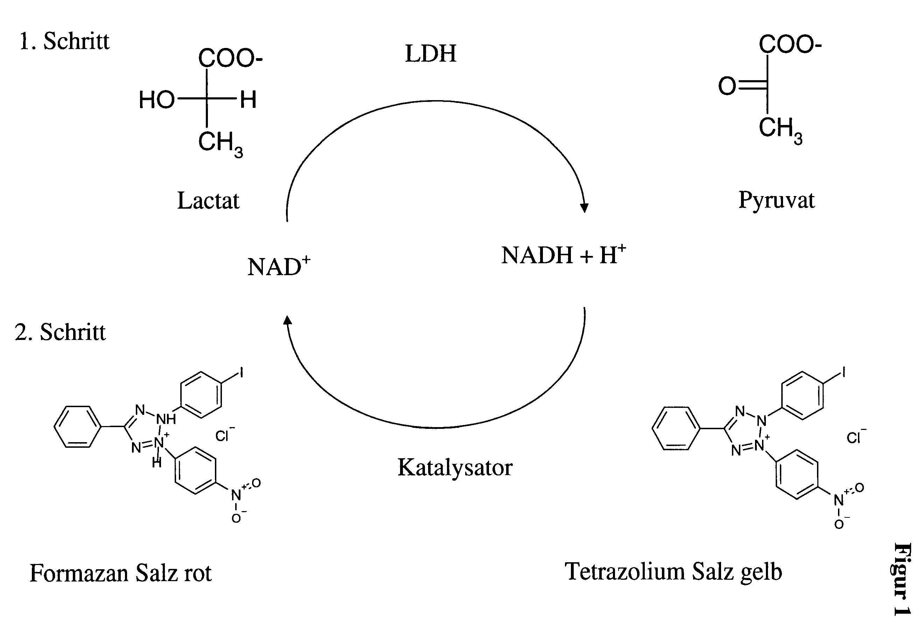

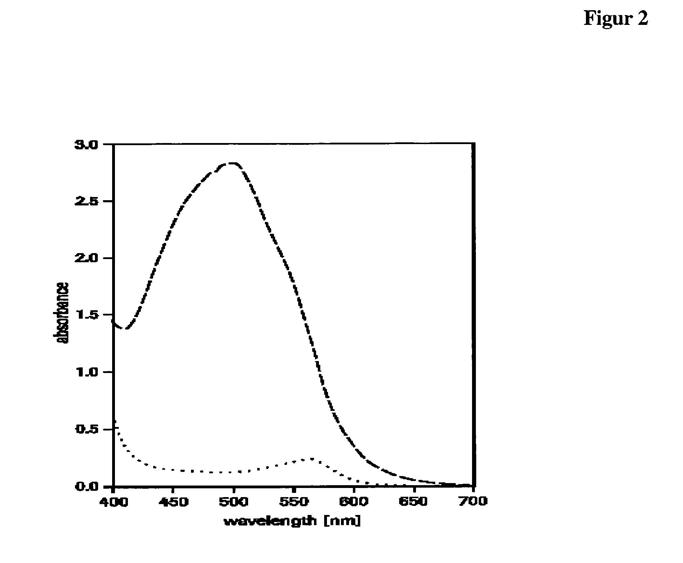

Bei diesem Test wird in einem ersten Schritt NAD+ zu NADH/H+ durch den LDHkatalysierte Umsetzung von Laktat zu Pyruvat reduziert. Im zweiten Schritt transferiert ein zweites Enzym (=Catalyst Diaphorase) H/H+ von NADH/H+ zum Tetrazolium Salz INT (2-[4-iodophenyl]-3-[4-nitrophenyl]-5-phenyltetrazolium chloride) welches dabei zum Formazan reduziert wird (Fig.1). Der Formazanfarbstoff ist wasserlöslich und zeigt ein breites Absorptionsmaximum bei 500 nm, während das Substrat INT bei dieser Wellenlänge nicht absorbiert (Fig. 2).

Bei anderen Methoden zur Zytotoxizitätsmessung werden auch in einem ähnlichen Reaktionsansatz statt colorimetrischen Enzymsubstraten fluoreszente Substrate wie Resazurin zur LDH- Messung (Cytotox-OneTM Assay der Firma Promega aus Madison, Wisconsin) oder zur Glucose-6-Phosphat Dehydrogenase Messung (Vybrant Assay der Firma Molecular Probes aus Eugene, Oregon) eingesetzt. Bei wieder anderen Methoden wird die freigesetzte Glycerinaldehyd-3-Phosphat Dehydrogenase zur Synthese von ATP über eine gekoppelte Enzymreaktion verwendet. Das dabei entstandene ATP wird in einer Biolumineszenzreaktion mit Luciferin und Luciferase eingesetzt, wobei ein messbares Lichtsignal entsteht (Corey, Michael J., et al., J. Immun. Meth. 207 (1997) 43-51).

Neben den Tests zur Messung einer zerstörten Plasmamembran wird häufig auch die physiologische Aktivität und Proliferationsrate der Zellen über die Fähigkeit bestimmte Farbsubstrate zu reduzieren ermittelt. Dabei werden Farbsubstrate eingesetzt, welche bei der Reduktion die Farbe in einer Weise ändert, dass nur das Produkt das Licht einer bestimmten Wellenlänge absorbiert, bei welcher dann gemessen wird. Bekannte Reagenzien hierfür sind beispielsweise Tetrazoliumsalze wie beispielsweise MTT (3-(4,5-dimethylthiazol-2-yl)-2,5-diphenyltetrazolium bromid), XTT (Natrium 3'-[(1-phenylamino-carbonyl)-3,4-tetrazolium]-bis(4-methoxy-6-nitro)-benzylsulfonsäure hydrat) und WST-1, welche von Roche Applied Science (Mannheim, Deutschland) unter den Namen Cell Proliferation Kit I (MTT), Cell Proliferation Kit II (XTT) und Cell Proliferation Reagent WST-1 angeboten werden.

Neben den Tetrazoliumsalzen wurde auch der blaue Farbstoff Resazurin verwendet, welcher sich bei der Reduktion in den roten, stark fluoreszenten Farbstoff Resorufin umsetzt, welcher sowohl colorimetrisch als auch fluorimetrisch gemessen werden kann (Lancaster et al., US Patent Nr. US 5,501,959).

WO 2003/089635 beschreibt eine Reagenzien-Kombination zur Bestimmung freigesetzter LDH mit den Substraten Resazurin, bzw. MTS (3-(4,5-dimethylthiazol-2-yl)-5-(3-carboxymethoxyphenyl)-2-(4-sulphophenyl)-2H-tetrazolium, inneres Salz). Dabei wird die reduzierte Form von MTS colorimetrisch und die reduzierte Form von Resazurin colorimetrisch oder bevorzugt fluorimetrisch gemessen. Bei dieser Reagenzienkombination wird auch eine Zugabe eines Stoppreagenzes vorgeschlagen, bei der die Reaktion abgestoppt wird. Vorgeschlagen als Stoppreagenz sind eine Seife, bzw. ein Detergenz oder eine starke Base wie beispielsweise NaOH. Als Seife, bzw. Detergenz wird eine 3 % SDS Lösung empfohlen. Dem Fachmann sind die Protein-denaturierenden Eigenschaften von SDS durchaus bekannt, wobei ein Stopp der Reaktion durch die Denaturierung der LDH eintritt. NaOH führt einen Reaktionsstopp durch die Änderungen des pH-Wertes herbei. Allerdings führen die wenigsten bekannten Detergenzien, oder Seifen zu einem Reaktionsstopp, wie die unten angegebenen Beispiele belegen. Die Lehre in WO 2003/089635 wäre also solche Detergenzien oder Seifen zu verwenden, welche einen Reaktionsstopp bewirken würden, also Protein-denaturierende Eigenschaften haben. Die in diesem Patent angegebenen Seifen oder Detergenzien enthalten zudem keinen Hinweis darauf, dass durch deren Verwendung eine Verringerung der Lichtstreuung oder Absorption verursacht durch Zellen vermieden und dadurch eine Erhöhung der Sensitivität colorimetrischer Messungen erreicht werden kann.

Bei allen Methoden, bei welchen eine Enzymaktivität in Gegenwart von Zellen colorimetrisch detektiert wird, gibt es insbesondere bei hohen Zellzahlen das Problem, dass das Licht im gesamten Wellenlängenbereich des sichtbaren Lichtes von den Zellen gestreut und absorbiert wird. So wird beispielsweise in der von Decker und Lohmann-Matthes beschriebenen Methode zur Messung von freigesetzter Lactatdehydrogenase darauf hingewiesen, dass man bei hohen Zellzahlen wegen der dabei beobachteten hohen Absorption der Zellen besser die zellfreien Überstände aus der Zellkulturplatte in neue Reaktionsgefäße überführt und dort die Messung der enzymatischen Aktivität durchführt. Dieses Vorgehen erfordert eine Zentrifugation der Kulturplatten und ein vorsichtiges Entfernen des Kulturüberstandes. Dies sind zusätzliche Arbeitsschritte, die eine Anwendung in der Hochdurchsatzanalyse beispielsweise mit entsprechenden Robotern erschweren. Zusätzlich können durch das Zentrifugieren oder das Abnehmen des Überstandes die Zellen beschädigt werden und dabei cytoplasmatische Enzyme freigesetzt werden, wodurch das Testergebnis gestört werden kann.

Bei den anderen Verfahren, bei denen eine fluorimetrische oder eine chemilumineszente Messung erfolgt kann man nicht mit den insbesondere für Standard Mikrotiterplatten weit verbreiteten colorimetrischen Messgeräten, den sogenannten ELISA Readern arbeiten, was die Nutzung dieser Substrate erheblich einschränkt. Bei den chemilumineszenten Substraten ist häufig eine relativ kurze Stabilität des Signals gegeben, was eine rasche Messung unmittelbar nach Ende des Testes erfordert.

In der in WO 2003/089635 veröffentlichten Methode zur Bestimmung freigesetzter LDH mit den Substraten Resazurin, bzw. MTS, werden diese auch für colorimetrische Messungen beschrieben. Als Stoppreagenz wird hierbei NaOH oder SDS beansprucht. Hierbei muss jedoch beachtet werden, dass die colorimetrische Messung des reduzierten Resazurin nur eine geringe Sensitivität erlaubt. Die Verwendung von NaOH oder SDS bei Tetrazoliumsalzen wie beispielsweise MTS ist von Nachteil, da NaOH zu einer Präzipitation von reduzierten Tetrazoliumsalzen führt (siehe Beispiel 2 dieser Anmeldung). Auch SDS ist nicht bevorzugt, da hierbei Tetrazoliumsalze überraschenderweise relativ schnell ausbleichen (siehe Beispiel 2 dieser Anmeldung).

### Kurzbeschreibung der Erfindung

Gegenstand der vorliegenden Erfindung ist deshalb ein Verfahren zur colorimetrischen Messung von Enzymaktivitäten, enthaltend folgende Schritte:
- Bereitstellen einer Zellsuspension oder eines Zellbestandteile enthaltenden Zell-Lysates
- Zugabe eines chromogenen Substrates zu Beginn der Enzymreaktion
- Zugabe einer ersten weiteren Substanz zum Abstoppen der Enzymreaktion,
- Colorimetrische Messung
welches erfindungsgemäß dadurch gekennzeichnet ist, dass vor, während oder nach Beendigung der Enzymreaktion eine zweite weitere Substanz zugesetzt wird, die die durch die Zellen oder die Zellbestandteile verursachte Lichtstreuung und oder Absorption reduzieren.

Dies kann beispielsweise dadurch erreicht werden, dass die zweite weitere Substanz eine Substanz ist, welche den Brechungsindex der Suspension oder des Lysates dem der Zellen oder Zellbestandteile angleicht.

Alternativ kann dies dadurch erreicht werden, dass die zweite weitere Substanz eine Substanz ist, die aufgrund ihrer molaren Konzentration durch osmotischen Druck den Zellen oder Zellbestandteilen Wasser entzieht.

Vorzugsweise kann dies auch dadurch erreicht werden, dass die zweite weitere Substanz die Zellmembran auflöst. Derartige Substanzen können bspw verschiedene Arten von Detergenzien, aber auch bestimmte Proteine oder Peptide darstellen.

Bestandteil der vorliegenden Erfindung sind darüber hinaus auch Kits zur colorimetrischen Messung von Enzymreaktionen in Zellsuspensionen oder Zellbestandteile enthaltenden Zell-Lysaten, enthaltend
- ein chromogenes Substrat
- eine erste Substanz zum Abstoppen der Enzymreaktion
- eine zweite Substanz, welche bei Zugabe die durch die Zellen oder die Zellbestandteile verursachte Lichtstreuung und/oder Absorption reduziert.

Beispielsweise kann die zweite Substanz erfindungsgemäß den Brechungsindex der Suspension oder des Zelllysates dem der Zellen oder Zellbestandteile angleichen.

Alternativ kann eine zweite weitere Substanz aufgrund ihrer molaren Konzentration durch osmotischen Druck den Zellen oder Zellbestandteilen Wasser entziehen.

Bevorzugterweise kann die zweite weitere Substanz Zellmembranen auflösen.

### Kurzbeschreibung der Abbildungen

### Figur 1

Im ersten Schritt reduziert ins Medium freigesetzte Lactat Dehydrogenase (LDH) NAD+ zu NADH+ H+ durch Oxidation von Lactat zu Pyruvat. In der zweiten enzymatischen Reaktion werden 2 H von NADH+ H+ auf das Tetrazolium Salz INT (2- [4-iodophenyl] -3- [4-nitrophenyl] -5-phenyltetrazolium chlorid) übertragen.

### Figur 2

Absorptionsspektrum der Working Solution aus dem Cytotoxicity Detection Kit (LDH). Die Reaction Mixture aus dem Cytotoxicity Detection Kit (LDH) wurde zu RPMI 1640 Medium mit 1 % Rinderserumalbumin (BSA) gegeben und das Absorptionsspektrum in Abwesenheit (.......) und Anwesenheit (-) von LDH gemessen.

### Detailierte Beschreibung der Erfindung

Die vorliegende Erfindung beruht auf einem Verfahren zur colorimetrischen Messung von Enzymaktivitäten in Gegenwart von Zellen, gekennzeichnet dadurch, dass zu den Zellen ein oder mehrere Substanzen zugesetzt werden, welche die durch die Zellen verursachte Lichtstreuung und Absorption minimieren.

Dies wird im Wesentlichen erreicht durch ein Verfahren, enthaltend die folgende Schritte
- Bereitstellen einer Zellsuspension oder eines Zellbestandteile enthaltenden Zell-Lysates
- Zugabe eines chromogenen Substrates zu Beginn der Enzymreaktion
- Zugabe einer ersten weiteren Substanz zum Abstoppen der Enzymreaktion,
- Colorimetrische Messung
wobei entweder vor, während oder nach Beendigung der Enzymreaktion eine zweite weitere Substanz zugesetzt wird, die die durch die Zellen oder die Zellbestandteile verursachte Lichtstreuung und/oder Absorption reduziert.

Üblicherweise muss bei hohen Zelldichten erst ein zellfreier Überstand durch Zentrifugation gebildet werden der dann nach dessen Abnahme vermessen werden kann. Die hier beschriebene Erfindung vermeidet diese zusätzlichen Arbeitsschritte dadurch, dass zu den Zellen während, oder am Ende des Testes, Reagenzien zugesetzt werden, welche die durch die Zellen verursachte Lichtstreuung und Absorption minimieren. Dies kann beispielsweise dadurch geschehen, dass der Brechungsindex der Zellen dem des Testmediums angeglichen wird und dadurch die Lichtstreuung reduziert bzw. minimiert wird. Dies kann beispielsweise durch eine Zugabe von Glycerin oder Ethylenglykol bis zu einer Endkonzentration von maximal 70 % erreicht werden.

In alternativen Ausführungsformen kann durch die Zugabe von Substanzen wie beispielsweise von Salzen in hoher molarer Konzentration das Volumen der Zellen und damit deren Absorption dadurch verringert werden, dass den Zellen durch den hohen osmotischen Druck des Mediums Wasser entzogen wird. Dadurch schrumpft das Volumen der Zellen, was eine Erhöhung der Durchsichtigkeit der zu testenden Probe zur folge hat. Beispielswiese hat sich in diesem Zusammenhang die Zugabe von mindestens 0.3 M NaCl als hinreichend erwiesen.

Bevorzugt für die Erniedrigung der Lichtstreuung und Absorption der Zellen ist die Permeabilisierung der Zellmembran. Dies kann beispielsweise durch lytische Enzyme wie beispielsweise Lipasen oder Mellitin erfolgen.

Bevorzugt geeignet zur Permeabilisierung sind aber Detergenzien. Dem Fachmann sind eine Vielfalt unterschiedlicher Detergenzien bekannt. Unterschieden wird hierbei zwischen ionischen, nicht-ionischen und zwitterionischen Detergenzien. Innerhalb der ionischen Detergenzien wird weiter zwischen anionischen Detergenzien wie beispielsweise SDS (Natriumdodecylsulfat), N-Laurylsarcosin oder Natriumcholat und kationischen Detergenzien wie beispielsweise Cetyltrimethylammonnium Bromid (CTAB), oder Dodecyltrimethylamonnium Bromid (DTAB) unterschieden. Beispiele für nicht-ionische Detergenzien sind dem Fachmann unter den Namen Triton®X-100 (Octylphenoxypolyethoxyethanol), Nonidet P40 oder TWEEN® 20 (Polyoxyethylen(20)sorbitan Monolaurat) bekannt. Gebräuchliche Zwitterionische Reagenzien sind beispielsweise CHAPS ([3-(3-Cholamidopropyl) dimethylammonio]-1-propansulfonsäure) oder Zwittergent®3-12 (n-Dodecyl-N,N dimethyl-3-ammonio-1-propansulfonsäure).

Überraschenderweise konnte mit den kationischen Detergenzien CTAB und DTAB eine Erhöhung der Absorption des reduzierten INT Substrates erreicht werden, was zu einer Sensitivitätssteigerung im Test führte. Zusätzlich waren die Signale mit diesen Detergenzien über einen Zeitraum von 5 Tagen sehr stabil, während mit reinem PBS-Puffer oder mit dem nicht-ionischen Detregenz TritonX100 eine deutliche Signalabnahme erfolgte und das anionische Detergenz SDS einen raschen Verlust der Absorption bewirkten.

Sämtliche Detergenzien werden erfindungsgemäß bis zu einer Endkonzentration bis maximal 3 % (v/v) und vorzugsweise bis maximal 1 % (v/v) zugesetzt.

Wird zur Erniedrigung der Lichtstreuung und Absorption der Zellen eine Permeabilisierung der Zellmembran durchgeführt, ist darauf zu achten, dass durch diesen Schritt freigesetzte Stoffe nicht die Reaktion beeinflussen. So könnten beispielsweise Enzyme freigesetzt werden, die weiteres Reaktionsprodukt herstellen, oder das Reaktionsprodukt weiter umsetzen, sodass es nicht mehr messbar ist. Es ist daher für einige enzymatische Reaktionen sinnvoll, gleichzeitig mit der Permeabilisierung der Zellen einen Stopp der Reaktion durchzuführen. Geeignete Maßnahmen hierfür sind abhängig von der zu messenden Reaktion.

Geeignete Massnahmen, um eine enzymatische Reaktion abzustoppen kann beispielsweise die Zugabe spezifischer Enzyminhibitoren, oder die Änderung der Reaktionsbedingungen in der Art sein, sodass die Reaktion beendet wird. In vielen Fällen ist die Änderung des pH-Wertes im Reaktionsmedium geeignet einen Reaktionsstopp zu bewirken. In den hier in den Beispielen untersuchten Fällen wurden NaOH und bevorzugt HCl zur Änderung des pH Wertes eingesetzt. HCl wurde auch im Cytotoxicity Detection Kit (LDH) (Roche Applied Science Cat. No. 11644793001) als Stoppreagenz für die LDH vorgeschlagen. Decker und Lohmann-Matthes (Decker, Thomas, und Lohmann-Matthes, Marie-Luise, J. Immun. Meth. 15 (1988) 61-69) schlagen in Ihrer Arbeit eine 15 mM Natriumoxamat Lösung zum Abstoppen von LDH als Inhibitor vor.

Darüber hinaus können die enzymatischen Reaktionen durch Zugabe von Protein-denaturierenden Stoffen wie beispielsweise Harnstoff oder durch Zugabe von Proteasen gestoppt werden.

Werden diese Substanzen durch deren Zugabe die durch Zellbestandteile verursachte Trübung der Probe minimiert wird, bereits vor Beginn oder während der Reaktion zugesetzt, so ist es erforderlich, dass diese Substanzen nicht die Enzymreaktion als solches beeinflussen.

Gegenstand der vorliegenden Erfindung ist auch die Verwendung von bestimmten Substanzen bei der colorimetrischen Messung von Enzymaktivitäten in Gegenwart von Zellen oder Zellbestandteilen, dadurch gekennzeichnet dadurch dass die verwendeten Substanzen die durch die Zellen oder Zellbestandteile verursachte Lichtstreuung und Absorption reduzieren und dabei die Enzymaktivitäten nicht beeinflusst werden.

Für eine derartige Verwendung haben sich die eingangs beschriebenen Proteine wie Lipasen oder Mellitin als geeignet erwiesen.

Besonders bevorzugt ist jedoch die Verwendung entsprechender Detergenzien wie N-Laurylsarcosin, Natriumcholat, Cetyltrimethylammonnium Bromid (CTAB), Dodecyltrimethylamonnium Bromid (DTAB), Triton®X-100 (Octylphenoxypolyethoxyethanol), Nonidet P40, TWEEN® 20 (Polyoxyethylen(20)sorbitan Monolaurat), CHAPS ([3-(3-Cholamidopropyl) dimethylammonio]-1-propansulfonsäure) und Zwittergent®3-12 (n-Dodecyl-N,N dimethyl-3-ammonio-1-propansulfonsäure). Alle geneannten Detergenzien werden erfindungsgemäß bis zu einer Endkonzentration bis maximal 3 % (v/v) und vorzugsweise bis maximal 1 % (v/v) verwendet.

Die vorliegende Erfindung betrifft auch Reagenzienkits, die die Durchführung der erfindungsgemäßen Verfahren ermöglichen. Reagenzienkits im Sinne der Erfindung können beispielsweise aus einem oder mehreren Substraten bestehen, von denen mindestens eines nach der enzymatische Umsetzung eine Farbänderung durchläuft, sodass entweder das Substrat, oder das Produkt dieser Reaktion bei einer bestimmten Wellenlänge spezifisch gemessen werden kann. Diese Substrate können entweder in einer gemeinsamen oder, falls dies eine höhere Stabilität gewährleistet, in unterschiedlichen Gefäßen Bestandteil des Reagenzienkits sein.

Gegenstand der Erfindung ist somit auch ein Kit zur colorimetrischen Messung von Enzymreaktionen in Zellsuspensionen oder Zellbestandteile enthaltenden Zell-Lysaten, enthaltend
- ein chromogenes Substrat
- eine erste Substanz zum Abstoppen der Enzymreaktion
- eine zweite Substanz, welche bei Zugabe die durch die Zellen oder die Zellbestandteile verursachte Lichtstreuung und/oder Absorption reduziert.

Beispielsweise kann die zweite weitere Substanz eine Substanz sein, welche den Brechungsindex der Suspension oder des Lysates dem der Zellen oder Zellbestandteile angleicht.

Alternativ kann die zweite weitere Substanz eine Substanz sein, die aufgrund ihrer molaren Konzentration durch osmotischen Druck den Zellen oder Zellbestandteilen Wasser entzieht.

Vorzugsweise kann die zweite weitere Substanz eine Substanz sein, die Zellmembran auflöst. Derartige Substanzen können bspw verschiedene Arten von Detergenzien sein, wie sie im Zusammenhang mit den erfindungsgemäßen Verfahren beschrieben wurden. Alternativ kann es sich bei den permeabilisierenden Substanzen um entsprechende Proteine oder Peptide wie beispielsweise Lipasen oder Mellitin handeln.

Zusätzliche typische Bestandteile eines Reagenzienkits sind Lösungen, die Puffersubstanzen und Salze optimiert für die jeweilige Enzymreaktion enthalten. Diese Lösungen werden üblicherweise zur Enzymreaktion zugesetzt. Diese Lösungen können entweder separat oder in Mischungen mit anderen Komponenten des Reagenzienkits angeboten werden. Viele Enzyme benötigen Coenzyme, welche ebenfalls Bestandteil des Reagenzienkits in separat abgefüllter oder gemeinsamer Formulierung mit andern Enzymen sein kann.

In vielen Fällen wird eine zu messende Enzymreaktion mit weiteren chemischen oder enzymatischen Reaktionen gekoppelt. Dabei wird dann entweder weiteres Substrat für die zu messende Reaktion erzeugt, oder das Reaktionsprodukt weiter umgesetzt. Ein Beispiel hierfür ist in Corey, Michael J., et al., J. Immun. Meth. 207 (1997) 43-51 zu finden, bei dem freigesetzte Glycerinaldehyd-3-Phosphat Dehydrogenase zur Synthese von ATP über eine gekoppelte Enzymreaktion verwendet. Das dabei entstandene ATP wird in einer Biolumineszenzreaktion mit Luciferin und Luciferase eingesetzt, wobei ein messbares Lichtsignal entsteht. Reagenzienkits Für derartige gekoppelte Reaktionen enthalten folglich zweckmäßigerweise weitere Enzyme, sowie Coenzyme, Reaktionspuffer und Substrate. Zusätzlich enthält der Reagenzienkits im Sinne der Erfindung mindestens eine Substanz, welche die durch die Zellen verursachte Lichtstreuung und Absorption minimieren und dabei die Enzymaktivität nicht beeinflussen. Diese Substanz kann in einer separaten Flasche vorhanden, oder beispielsweise Bestandteil eines Reaktionspuffers sein. In Fällen, in denen ein cytoplasmatisches Enzym gemessen wird und zur Minimierung der durch die Zellen verursachten Lichtstreuung und Absorption eine Permeabilisierung der Zellmembran angewendet wird, enthält der Reagenzienkit sinnvollerweise zusätzlich eine Substanz, welche die Farbreaktion abstoppt. Diese Komponente kann in einer gemeinsamen Formulierung mit einer anderen Komponente des Reagenzienkits wie beispielsweise der Substanz zur Permeabilisierung der Zellmembran oder separater Bestandteil des Reagenzienkits sein.

Die folgenden Beispiele, Publikationen und Abbildungen erläutern die Erfindung, deren Schutzumfang sich aus den Patentansprüchen ergibt, weiter. Die beschriebenen Verfahren sind als Beispiele zu verstehen, die auch noch nach Modifikationen den Gegenstand der Erfindung beschreiben.

### Beispiele

### Beispiel 1,

**Messung der Absorption von Zellen nach Behandlung mit unterschiedlichen Reagenzien.**

Die humane Suspensionszelllinie U 937 wurde in RPMI 1640 Medium (Firma Sigma) mit 10 % fötalem Kälberserum und 2 mM Glutamine angezüchtet. Die Zellen wurden dann in frischem RPMI Medium gewaschen und dabei auf 25 000, 50 000, bzw. 100 000 Zellen/100 µl eingestellt. Davon wurde jeweils 100 µl in eine 96 Loch Standard Mikrotiterplatte übertragen. Zu den Zelllösungen wurde jeweils 50 µl RPMI Medium mit den entsprechenden Reagenzien pipettiert.

Nach 5 min und 75 min Inkubation wurde jeweils die Absorption bei 690 nm Wellenlänge gemessen.

In der folgenden Tabelle erkennt man, dass die Absorption der Zellen mit zunehmender Zellzahl steigt. Außerdem wird deutlich, dass alle Reagenzien die Absorption gegenüber dem reinen Medium senken, wobei die optimalen Einsatzkonzentrationen noch zu ermitteln sind.

| Reagenz | Zellzahl | Absorption nach 5 min | Absorption nach 75 min |
|---|---|---|---|
| Triton X100 ad 1 % (V/V) | 25 000 Zellen | 0,001 | 0,001 |
| | | | |
| | 50 000 Zellen | 0,004 | 0,004 |
| | | | |
| | 100 000 Zellen | 0,008 | 0,008 |
| SDS ad 1 % (V/V) (Stand der Technik) | 25 000 Zellen | 0,002 | 0,003 |
| | | | |
| | 50 000 Zellen | 0,006 | 0,006 |
| | | | |
| | 100 000 Zellen | 0,010 | 0,010 |
| CTAB ad 1 % (V/V) | 25 000 Zellen | 0,006 | 0,003 |
| | | | |
| | 50 000 Zellen | 0,031 | 0,014 |
| | | | |
| | 100 000 Zellen | 0,025 | 0,018 |
| DTAB ad 1 % (V/V) | 25 000 Zellen | 0,005 | 0,006 |
| | | | |
| | 50 000 Zellen | 0,012 | 0,011 |
| | | | |
| | 100 000 Zellen | 0,024 | 0,021 |
| N-Laurylsarcosin ad 1 % (V/V) | 25 000 Zellen | 0,001 | 0,002 |
| | | | |
| | 50 000 Zellen | 0,003 | 0,004 |
| | | | |
| | 100 000 Zellen | 0,012 | 0,013 |
| RPMI (negative Kontrolle) | 25 000 Zellen | 0,01 | 0,011 |
| | | | |
| | 50 000 Zellen | 0,018 | 0,018 |
| | | | |
| | 100 000 Zellen | 0,044 | 0,048 |

### Beispiel 2.

**Abstoppen der LDH Reaktion mit verschiedenen Stoppreagenzien.**

Für diesen Versuch werden zunächst Lysate von U 937 Zellen als Quelle für LDH hergestellt. Dazu wurden 6x105 Zellen in 1 ml destilliertem Wasser suspendiert und mit Ultraschall behandelt, bis alle Zellen lysiert waren. Anschließend wurde das Lysat für 10 min. bei 200 xg zentrifugiert und der Überstand frei von Zellresten für weitere Versuche verwendet.

Zellfreie Lysate von je 5000 Zellen wurde in einen LDH Test mit Komponenten aus dem Cytotoxicity Detection Kit (LDH) (Roche Applied Science Cat. No: 11644793) eingesetzt.

Abweichend von dem erwähnten Kit wurde eine Lösung bestehend aus 2 mM INT, 90 mM L-Lactat, 100 mM Tris, pH 8,5 ohne Detergenz als "Dye Solution" eingesetzt.

Nach einer Reaktionszeit von 5 min wurden zu diesen Ansätzen unterschiedliche Stoppreagenzien zugesetzt und die Absorption bei 492 nm (dem Absorptionsmaximum von reduzierten INT) verfolgt.

| Stoppreagenz | Absorption 0 min nach Stoppreagenz Zugabe | Absorption 30 min nach Stoppreagenz Zugabe | Absorption 120 min nach Stoppreagenz Zugabe |
|---|---|---|---|
| RPMI Medium | 0,113 | 0,309 | 0,852 |
| (negative Kontrolle) | | | |
| SDS ad 3 % (V/V) | 0,113 | 0,113 | 0,105 |
| (Stand der Technik) | | | |
| 15 mM Natriumoxamat | 0,113 | 0,127 | 0,179 |
| 15 mM Natriumoxamat + Triton X100 ad 1 % (V/V) | 0,113 | 0,127 | 0,191 |

In der Tabelle erkennt man, dass sowohl SDS, als auch Natriumoxamat die LDH Reaktion stoppen. Allerdings gelingt der Stopp mit der angewendeten Oxamat-Konzentration nicht zu 100 %, wie das bei SDS der Fall ist. Eine Optimierung der Oxamat-Konzentration wäre hier nötig. Das Beispiel mit der Kombination von Oxamat mit Triton X100 zeigt, dass dieser Inhibitor auch in Gegenwart dieses Detergenzes aktiv ist. Bei der Verwendung von SDS ist eine leichte Abnahme der Absorption zu beobachten, was auf eine Instabilität des Substrates in Kombination mit SDS deutet.

In einem weiteren Versuch wurde HCl und NaOH als Stoppreagenz eingesetzt

Hierfür wurden zellfreie Lysate von je 1000 Zellen in einen LDH Test mit Komponenten aus dem Cytotoxicity Detection Kit (LDH) mit der modifizierten "Dye Solution" eingesetzt. Nach einer Reaktionszeit von 30 min wurden zu diesen Ansätzen unterschiedliche Stoppreagenzien zugesetzt und die Absorption bei 492 nm (dem Absorptionsmaximum von reduzierten INT) verfolgt.

| Stoppreagenz | Absorption 0 min nach Stoppreagenz Zugabe | Absorption 30 min nach Stoppreagenz Zugabe | Absorption 120 min nach Stoppreagenz Zugabe |
|---|---|---|---|
| RPMI Medium | 0,280 | 0,448 | 0,962 |
| 0,2 M HCl | 0,275 | 0,271 | 0,261 |
| | | | |
| 0,2 M HCl | 0,268 | 0,269 | 0,261 |
| + Triton X100 ad 1 % (V/V) | | | |

Hier wird erkennbar, dass HCl ein geeignetes Stoppreagenz für die LDH Reaktion darstellt. Die Kombination von HCl mit Triton X100 führt hier zum gleichen Ergebnis.

Die Zugabe von NaOH in 0,2 M Endkonzentration führte zur sofortigen Präzipitation des Farbstoffes.

### Beispiel 3

**Messung unterschiedlicher Mengen freigesetzter LDH in Gegenwart hoher Zellmengen.**

Zellfreie Lysate von 50 000, 10 000 und 1000 U 937 Zellen wurden in 50 µl Volumen in den Vertiefungen einer Standardmikrotiterplatte für 0, 5, bzw. 30 Minuten in einem LDH Test mit 100 µl Reaktionslösung aus dem Cytotoxicity Detection Kit (LDH) mit modifizierter "Dye Solution" bei Raumtemperatur inkubiert. Nach 5, bzw. 30 Minuten Inkubationszeit wurde mit 6.5 µl HCl 25 %-ig die Reaktion abgestoppt. Anschließend wurden zu diesen Ansätzen jeweils 50 µl mit 150 000 U937 Zellen und gleichzeitig 50 µl verschiedener Detergenzienlösungen zugegeben.

Anschließend wurde bei einer Wellenlänge von 492 nm jeweils nach 10 Minuten und nach 5 Tagen gemessen. Die Leerwerte eines Kontrollansatzes, bei welchem die Reaktion sofort nach 0 Minuten abgestoppt wurden, wurden jeweils von den Messwerten subtrahiert.

| Reagenz | Lysatmenge (Zellzahl) | Absorption nach 5 min | Absorption nach 30 min |
|---|---|---|---|
| | | Messung 10 min nach Abstoppen der Reaktion | Messung 10 min nach Abstoppen der Reaktion |
| | | (Messung nach 5 Tagen) | (Messung nach 5 Tagen) |
| PBS | 50 000 Zellen | 0,048 (0,024) | 0,288 (0,119) |
| | | | |
| (Phosphatpuffer mit Salzen) | 10 000 Zellen | 0,000 (0,000) | 0,067 (0,033) |
| | | | |
| | 1 000 Zellen | 0,000 (0,000) | 0,000 (0,000) |
| (negative Kontrolle) | 0 Zellen | 0,000 (0,000) | 0,000 (0,000) |
| Triton X100 ad 1 % (V/V) | 50 000 Zellen | 0,101 (0,093) | 0,338 (0,150) |
| | | | |
| | 10 000 Zellen | 0,038 (0,035) | 0,078 (0,065) |
| | | | |
| | 1 000 Zellen | 0,003 (0,001) | 0,006 (0,001) |
| | | | |
| | 0 Zellen | 0,000 (0,000) | 0,000 (0,000) |
| SDS ad 1 % (V/V) (Stand der Technik) | 50 000 Zellen | 0,093 (0,008) | 0,364 (0,021) |
| | | | |
| | 10 000 Zellen | 0,011 (0,000) | 0,078 (0,000) |
| | | | |
| | 1000 Zellen | 0,000 (0,000) | 0,007 (0,000) |
| | | | |
| | 0 Zellen | 0,000 (0,000) | 0,000 (0,000) |
| CTAB ad 1 % (V/V) | 50 000 Zellen | 0,095 (0,085) | 0,372 (0,363) |
| | | | |
| | 10 000 Zellen | 0,036 (0,021) | 0,084 (0,077) |
| | | | |
| | 1 000 Zellen | 0,001 (0,002) | 0,010 (0,004) |
| | | | |
| | 0 Zellen | 0,000 (0,000) | 0,000 (0,000) |
| DTAB ad 1 % (V/V) | 50 000 Zellen | 0,088 (0,082) | 0,370 (0,299) |
| | | | |
| | 10 000 Zellen | 0,034 (0,026) | 0,075 (0,069) |
| | | | |
| | 1 000 Zellen | 0,002 (0,002) | 0,008 (0,005) |
| | | | |
| | 0 Zellen | 0,000 (0,000) | 0,000 (0,000) |

Man erkennt, dass man durch die Zugabe der Detergenzien die LDH aus dem Lysat von 10000 Zellen bereits nach 5 min Reaktionszeit in Gegenwart von 150 000 ganzen Zellen nachweisen kann, während dies bei Verwendung reinen PBS Puffers ohne Detergenz nicht möglich ist. Nach 30 min Reaktionszeit kann bereits die LDH aus 1000 Zellen in diesem Hintergrund in den Detergenz-behandelten Proben nachgewiesen werden, während ohne Detergenz nur 10000 Zellen detektierbar sind. Dies liegt daran, dass bei den nicht Detergenz-behandelten Proben eine höhere Hintergrund-Absorption verursacht durch die 150 000 Zellen vorhanden ist.

Überraschenderweise kann man feststellen, dass für dieses Farbsubstrat kationische Detergenzien wie CTAB und DTAB die Messwerte über 5 Tage nahezu stabil halten, während SDS hier destabilisierend wirkt.

### Referenzliste

Corey, Michael J., et al., J. Immun. Meth. 207 (1997) 43-51
Decker, Thomas, und Lohmann-Matthes, Marie-Luise, J. Immun. Meth. 15 (1988) 61-69
Korzeniewski, Carol, und Callewaert, Denis M., J. Immun. Meth. 64 (1983) 313-320 Martin, Angela, und Clynes, Martin, In Vitro Cell Dev. Biol. 27A (1991) 183-184 Masanet, J., Gomez-Lechon, M. J., und Castell, J. V., Toxic. in Vitro 2 (1988) 275-282
Szekeres, Julia, Pacsa, A.S., und Pejtsik, B., J. Immun. Meth. 40 (1981) 151-154 US 5,501,959
WO 2003/089635

## Patentansprüche

1. Verfahren zur colorimetrischen Messung einer Enzymreaktion, enthaltend folgende Schritte
- Bereitstellen einer Zellsuspension oder eines Zellbestandteile enthaltenden Zell-Lysates
- Zugabe eines chromogenen Substrates zu Beginn der Enzymreaktion
- Zugabe einer ersten weiteren Substanz zum Abstoppen der Enzymreaktion, Colorimetrische Messung
**dadurch gekennzeichnet, dass** vor, während oder nach Beendigung der Enzymreaktion eine zweite weitere Substanz zugesetzt wird, die die durch die Zellen oder die Zellbestandteile verursachte Lichtstreuung und/oder Absorption reduziert.

2. Verfahren nach Anspruch 1, **dadurch gekennzeichnet, dass** die zweite weitere Substanz den Brechungsindex der Suspension oder des Zell-Lysates dem der Zellen oder Zellbestandteile angleicht.

3. Verfahren nach Anspruch 1, **dadurch gekennzeichnet, dass** die zweite weitere Substanz aufgrund ihrer molaren Konzentration durch osmotischen Druck den Zellen oder Zellbestandteilen Wasser entzieht.

4. Verfahren nach Anspruch 1-2 **dadurch gekennzeichnet, dass** die zweite weitere Substanz die Zellmembran auflöst.

5. Verfahren nach Anspruch 4 **dadurch gekennzeichnet, dass** die zweite weitere Substanz ein Protein oder Peptid ist.

6. Verfahren nach Anspruch 4 **dadurch gekennzeichnet, dass** die zweite weitere Substanz ein Detergenz ist.

7. Verwendung von Substanzen, welche die durch Zellen oder Zellbestandteile verursachte Lichtstreuung und Absorption reduzieren und dabei die Enzymaktivitäten nicht beeinflussen
als Additiv bei der corlorimetrischen Messung von Enzymaktivitäten in Gegenwart von Zellen oder Zellbestandteilen.

8. Kit zur colorimetrischen Messung von Enzymreaktionen in Zellsuspensionen oder Zellbestandteile enthaltenden Zell-Lysaten, enthaltend
- ein chromogenes Substrat
- eine erste Substanz zum Abstoppen der Enzymreaktion
- eine zweite Substanz, welche bei Zugabe die durch die Zellen oder die Zellbestandteile verursachte Lichtstreuung und/oder Absorption reduziert.

9. Kit nach Anspruch 7 **dadurch gekennzeichnet, dass** die zweite Substanz den Brechungsindex der Suspension oder des Zelllysates dem der Zellen oder Zellbestandteile angleicht.

10. Kit nach Anspruch 7-8 **dadurch gekennzeichnet, dass** die zweite weitere Substanz die Zellmembran auflöst.
